# EUROPEAN PATENT SPECIFICATION

(11) **EP 2 381 949 B1**
(45) Date of publication and mention of the grant of the patent: **21.11.2012**
(21) Application number: 09798927.1
(22) Date of filing: 22.12.2009
(51) Int. Cl.: A61K 31/704, A61K 31/7088, A61K 39/39, A61P 35/00

(54) **IMMUNOSTIMULATING SAPONINS FOR USE IN IN SITU TUMOR-DESTRUCTION THERAPY**
IMMUNSTIMULIERENDE SAPONINE FÜR IN-SITU-TUMORDESTRUKTIONSTHERAPIE
SAPONINES IMMUNOSTIMULANTES S'UTILISANT DANS LE TRAITEMENT DE DESTRUCTION TUMORALE IN SITU

(30) Priority: 23.12.2008 EP 08172775
(43) Date of publication of application: 02.11.2011
(73) Proprietor: Intervet International BV, 5831 AN Boxmeer (NL); Stichting Katholieke Universiteit, Universitair Medisch Centrum St Radboud, 6525 EZ Nijmegen (NL)
(72) Inventor: SCHRIER, Carla Christina, NL-5831 AN Boxmeer (NL); NIERKENS, Stefan, NL-6500 HB Nijmegen (NL); ADEMA, Gosse, NL-6500 HB Nijmegen (NL); BROK den, Martijn, NL-6500 HB Nijmegen (NL); RUERS, Theodoor Jaques Marie, NL-3707 EE Zeist (NL)
(74) Representative: Keus, Jacobus Albertus Ronald
(86) International application number: PCT/EP2009/067721
(87) International publication number: WO 2010/072743

(56) References cited:
- US-A1- 2005 175 623
- BARR I G ET AL: "ISCOMS AND OTHER SAPONIN BASED ADJUVANTS" ADVANCED DRUG DELIVERY REVIEWS, ELSEVIER BV, AMSTERDAM, NL LNKD- DOI:10.1016/S0169-409X(98)00013-1, vol. 32, no. 3, 6 July 1998 (1998-07-06), pages 247-271, XP009052660 ISSN: 0169-409X
- LENARCZYK A ET AL: "ISCOM<(>R) based vaccines for cancer immunotherapy" VACCINE, ELSEVIER LTD, GB LNKD- DOI:10.1016/J.VACCINE.2003.09.014, vol. 22, no. 8, 25 February 2004 (2004-02-25), pages 963-974, XP004489106 ISSN: 0264-410X
- CHAUNG ET AL: "CpG oligodeoxynucleotides as DNA adjuvants in vertebrates and their applications in immunotherapy" INTERNATIONAL IMMUNOPHARMACOLOGY, ELSEVIER, AMSTERDAM, NL LNKD- DOI:10.1016/J.INTIMP.2006.06.001, vol. 6, no. 10, 1 October 2006 (2006-10-01), pages 1586-1596, XP024976928 ISSN: 1567-5769 [retrieved on 2006-10-01]
- DEN BROK MARTIJN H M G M ET AL: "Synergy between in situ cryoablation and TLR9 stimulation results in a highly effective in vivo dendritic cell vaccine" CANCER RESEARCH, vol. 66, no. 14, July 2006 (2006-07), pages 7285-7292, XP002579190 ISSN: 0008-5472
- STEPHAN ROUX ET AL: "Tumor destruction using electrochemotherapy followed by CpG oligodeoxynucleotide injection induces distant tumor responses" CANCER IMMUNOLOGY, IMMUNOTHERAPY, SPRINGER, BERLIN, DE, vol. 57, no. 9, 8 February 2008 (2008-02-08), pages 1291-1300, XP019624386 ISSN: 1432-0851 cited in the application
- JAHRSDORFER ET AL: "CpG oligodeoxynucleotides as immunotherapy in cancer" UPDATE ON CANCER THERAPEUTICS, ELSEVIER, AMSTERDAM, NL LNKD- DOI:10.1016/J.UCT.2007.11.003, vol. 3, no. 1, 11 January 2008 (2008-01-11), pages 27-32, XP022547504 ISSN: 1872-115X

## Description

The present invention relates to pharmaceutical compositions for use in *in situ* tumor-destruction therapy comprising the steps of tumor destruction and administration of an immunostimulating amount of an immunopotentiaton that is contained in the pharmaceutical composition, and to the use of such pharmaceutical compositions in the manufacture of a medicament.

Cancer is a general term, used to describe neoplastic growth. Neoplasms are considered abnormal, usually de-differentiated forms of tissue that commonly proliferate at a higher speed than normal. In most cases neoplastic cells invade surrounding tissue and moreover they metastasize and continue to grow elsewhere in the body.

Local and regional treatment of the neoplastic mass, the tumor, such as surgery, does not affect possible metastases. Therefore, additional therapies are needed such as treatment with cytotoxic drugs. Such treatment is generally known as chemotherapy.

Local treatment is of course a first step in the treatment of solid tumors. This is traditionally done by means of tumor resection.

Another approach is tumor destruction *in situ.* A characteristic of tumor destruction *in situ* is that the tumor is not removed but necrotized. In principle irradiation is a form of tumor destruction *in situ,* but many other ways of tumor destruction have been developed. Common methods are e.g. photodynamic therapy using the combination of photosensitizing compounds and their subsequent activation by laser, *in situ* heating by means of laser light, microwaves, electric current, ultrasound, high intensity focused ultrasound or by means of radiofrequency waves, or cryotherapy: necrotizing tissue by freezing.

Tumor destruction *in situ* leaves the destructed tumor mass present in the body. This leaves the possibility open to try and build an immunological response to tumor-specific antigens (cancer immunotherapy). The advantage of successful induction of such an immunological response to tumor-specific antigens is that it will last for some time and eventually eliminate tumor localization elsewhere in the body that is not amenable for local tumor destruction.

However, contrary to what is known from vaccine development that is based upon non-self antigens, the induction of an immune response against tumor antigens is far from easy.

Basically, tumor antigens are predominantly normal components of the body: self-antigens. Therefore the immune system as such will down-regulate self-directed immune response leading to a tolerant state for self-antigens.

Thus, the development of tumor destruction-based cancer immunotherapy requires a very specific approach.

Actually, the non-methylated cytidyl guanosyl oligodeoxynucleotides (CpG ODN) are currently considered to be the by far most preferred specific group of immunopotentiating compounds capable of inducing an immune response against tumor-specific self-antigens.

These cytidyl guanosyl oligodeoxynucleotides act as toll-like receptor 9 (TLR9) agonists. CpG motifs stand out because of their preferential induction of Th1 responses and tumor-specific CD8⁺ T lymphocytes. TLR9 is predominantly expressed by B cells and dendritic cells (DC) that internalize and directly respond to CpG motifs. Upon triggering of TLR9, DCs mature and migrate to draining lymph nodes where they present antigens to T and B lymphocytes. Importantly, these DCs acquire the unique ability to present captured antigens on MHC class I molecules, a process known as cross-presentation, which is crucial for efficient priming of tumor-specific CTLs. As such, CpG administration has been reported to prevent tumor outgrowth in a prophylactic setting and could also eradicate established tumors in mice. Nierkens, S. et al. (Cancer Res. 68: 5390-5396 (2008)) and by Roux, S. et al. (Cancer Immunol. Immunoth. 57: 1291-1300 (2008))

There are however some potential safety concerns with regard to the use of CpG ODNs, that i.a. include the induction of anti-DNA antibodies and autoimmunity. Furthermore, their toxicity when given in both higher amounts and over a longer period of time, as well as the costs involved in their use are of concern.

Therefore, there is a need for other immunopotentiating compounds.

The present invention provides means to decrease or overcome the concerns mentioned above.

Given the key role of TLR9 and its agonists in the induction of an immunological response to tumor-specific antigens after tumor destruction *in situ,* the skilled person would consider it a prerequisite that such other immunopotentiating compounds also act as TLR9 agonists. Surprisingly it was found now that saponins, that bear no relation to the TLR9-mechanism at all, are nevertheless very suitable for inducing an immunological response to tumor-specific self-antigens after tumor destruction. Even more unexpectedly, their effectivity, although through unknown mechanisms, appears to be comparable to, or even better than that of CpGs.

It was found that the administration of saponins in or around a tumor, at or around the moment of tumor destruction induces a very significant immunological response to tumor-specific antigens after tumor destruction *in situ.* This immunological response is long-lasting and is therefore very suitable to eliminate metastasized cells, even if such cells have been latently present in the body. Moreover, this immunological response appeared to be sufficiently strong to prevent the multiplication of the same type of tumor cells even if these are deliberately administered in substantial amounts several weeks after the treatment.

Saponins have up till now only been described as adjuvants against non-self antigens; e.g. in bacterial or viral vaccines. The use of saponins as cytotoxins, for the killing of tumor cells, has been described by Bachran, C. et al. (Medicinal Chemistry 8: 575-584 (2008)). In PCT-application WO 2008/063129, the use of saponins in lipid-containing particles, as cytotoxins for the killing of tumor cells is described.

However in the present invention their cytotoxic effect is of no relevance, as the saponin is used in combination with cells that are already killed in the process of tumor destruction. Since their cytotoxic effect plays no role, no effect on the destructed tumor would be expected anyway. Moreover the cytotoxic effect of saponins in chemotherapy only works at the moment of administration. They do not build an immunological response and thus they do not act against metastasized cells that temporarily have low metabolic activity; latent cells.

The role of saponins in inducing an immunological response to tumor-specific self-antigens, following tumor destruction *in situ* was hitherto unknown and could for the reasons mentioned above not be expected.

Therefore, a first embodiment of the invention relates to pharmaceutical compositions for use in *in situ* tumor-destruction therapy comprising the steps of tumor destruction and administration of an immunostimulating amount of an immunopotentiaton that is contained in the pharmaceutical composition, wherein that said immunopotentiator is a saponin.

Saponin is in principle the generic name for a group of plant glycosides, of which the *Quillaja saponaria* saponin is the oldest and most frequently used.

Crude saponin actually is a mixture of saponins sharing a same basic structure, but having different side chains. The different saponin components differ mainly in their degree of hydrophilicity/phobicity.

HPLC is a preferred method to detect and isolate the various saponin components from a crude saponin mixture. Several purified extracts such as QS-7, -17, -18, -21, GPI-0100, QuilA, Qvac and BioQ are commercially available from various sources.

Preferably, saponin comprises at least one of the following components: QS-7, QS-17, QS-18 or QS-21.

Also preferred saponins are QuilA and components thereof, Vax Sap, SuperSap, GPI-0100, QP UF 1000 and the like.

Thus, a preferred form of this embodiment relates to a pharmaceutical composition for use according to the invention, wherein the saponin comprises at least one of the following components: QS-7, QS-17, QS-18, QS-21, QuilA, Vax Sap, SuperSap, GPI-0100 or QP UF 1000.

Another attractive immunostimulating form of saponin are so-called empty immune stimulating complexes (empty ISCOMS). Empty immune stimulating complex preparations differ from saponin in that they are made from a mixture of saponin, lipid and cholesterol. During their preparation small micelle-like particles are formed, that are even more immunopotentiating than saponin as such.

Thus, another preferred form of this embodiment relates to a pharmaceutical composition for use according to the invention wherein the saponin is in the form of an empty immune stimulating complex.

It goes without saying that the present invention is equally applicable in the field of human and veterinary medicine.

In view of the different modes of action of saponins on the one hand and CpG ODNs on the other hand, one could not expect any enhancing effect of the combined administration of the two. Surprisingly, however, it was found that there is a strong synergistic effect in the combined use of saponin and CpG ODNs.

This unexpected synergy is advantageous, because this makes it possible to use sub-standard amounts of CpG ODNs when administered in combination with saponin. This in turn clearly diminishes the disadvantages of the use of CpG ODNs that were mentioned above. In this light, the use of CpG ODNs becomes attractive again, provided that they are given in combination with saponin.

The use of saponin in combination with CpG has been described as an adjuvant for the induction of an immune response against non-self antigens in US Patent US 7049302, but for the reasons set out above a combined effect, let alone a synergistic effect, could be expected against self-antigens.

Thus, a more preferred form of this embodiment relates to the pharmaceutical composition for use according to the invention that in addition comprises CpG ODNs.

CpG ODNs for use in immune stimulation have been described since 1994 (US Patent US6429199). CpG-motifs basically have the structure 5'-X₁-C-pG-X₂-3'. The CpG motif 5'-Pu-Pu-CpG-Pyr-Pyr is known to be amongst the most immunopotentiating (Scheule, R.K., Advanced Drug Delivery Reviews 44: 119-134 (2000)). Basically, their length is from 8-80 bases and they contain at least one non-methylated CpG-motif.

Small differences in efficiency in different animal species are frequently seen. Merely as an example; human TLR9 is optimally triggered by the CpG motif G-T-CpG-T-T, whereas mouse TLR9 is more optimally triggered by G-A-CpG-T-T (Krieg, A.M., Nature Medicine 9: 831-835 (2003).

Optimal CpG motifs for seven veterinary and three laboratory species have been described by Rankin, R., et al., in Antisense and Nucleic Acid Drug Development 11: 333-340 (2001). CpG-motifs that efficiently stimulate canine and feline immune cell proliferation are described by Wernette, C.M., et al., in Veterinary Immunol. And Immunopath. 84: 223-236 (2002). Applications for CpG-motifs in poultry have been described i.a. by Ameiss, K.A., et al., in Veterinary Immunol. And Immunopath. 110: 257-267 (2006).

CpG ODNs with different CpG-motives are easily commercially available, and if desired they are easily synthesized. Suitable amounts of CpG ODNs can be found i.a. in the publications mentioned above and in the Examples section.

Again, as mentioned above, it goes without saying that the present invention is equally applicable in the field of human and veterinary medicine, although it is advisable (though not mandatory) to match the CpG-motif used to the animal species for which the invention is used. This can easily be done on the basis of the publications summarized above.

In principle, the steps of tumor destruction and administration of an immunopotentiator can be performed at different moments in time or at the same time. Theoretically, however, one would expect that conditioning a tumor with the immunopotentiator several days or better a week or even two or more weeks before applying tumor destruction, with the aim of "priming" the immune system, would be the preferred route.

Surprisingly however, it was found that if the administration of the immunopotentiator is done after tumor destruction, within days after tumor destruction, preferably within one day, more preferably within 12 hours, even more preferably within 6 hours, still even more preferably within 2 hours after tumor destruction, the level of immunostimulation is better than when the order of the steps is reversed.

Also very good results are obtained when the administration of the immunopotentiator is done between about two hours before the tumor destruction and the moment of destruction. This is because after destruction the neoplastic mass may be more difficult to approach or enter due to destruction-induced changes in its structure.

Administration of the immunopotentiator in the interval between 2 hours before and two hours after tumor destruction is called peri-operative administration.

Therefore, one preferred form of this embodiment relates to a pharmaceutical composition for use in *in situ* tumor-destruction therapy comprising the steps of tumor destruction and administration of an immunopotentiator according to the invention, wherein said steps are in the following order:
a. destruction of the tumor
b. administration of an immunopotentiator.

More preferred forms of this embodiment relate to the steps in the order as mentioned above wherein the administration of an immunopotentiator follows within 24 hours, 12 hours or even 6 hours after tumor destruction, in that order of preference.

Also, another preferred form of this embodiment relates to a pharmaceutical composition for use in *in situ* tumor-destruction therapy comprising the steps of tumor destruction and administration of an immunopotentiator according to the invention, wherein said steps are:
a. Peri-operative administration of the immunopotentiator
b. destruction of the tumor

With regard to the site or sites of administration of the immunopotentiator, the following considerations should be made:
Preferably, the immunopotentiator is administrated directly into the neoplastic mass. Although slightly less preferred, peri-tumoral administration where the immunopotentiator is administered at one or more locations around the neoplastic mass is also possible. Another, though less preferred administration is subcutaneous administration in the draining area of the neoplastic mass. Finally, intravenous administration, preferably close to the location of the neoplastic mass is possible.

Therefore, the said administration of the immunopotentiator takes place by intravenous administration, subcutaneous administration in the draining area of the neoplastic mass, peri-tumoral administration or intra-tumoral administration, in that order of increasing preference.

Another embodiment of the present invention relates to the use of a pharmaceutical composition according to the invention in the manufacture of a medicament for the treatment of a mammal suffering from cancer wherein the mammal has been subjected to tumor destruction.

Still another embodiment of the present invention relates to the use of a pharmaceutical composition according to the invention in the manufacture of a medicament for peri-operative administration, for the treatment of a mammal suffering from cancer wherein the mammal will be or has been subjected to tumor destruction.

### Examples

### Example 1

### Mice and tumor cells

C57BL/6n mice (6-8 weeks old) were purchased from Charles River Wiga (Sulzfeld, Germany) and maintained under specific pathogen-free barrier conditions at the Central Animal Laboratory (Nijmegen, The Netherlands). Drinking water and standard laboratory food pellets were provided *ad libitum* and mice were allowed to settle for at least 1 week before random assignment into specific treatment groups. The experiments were performed according to the guidelines for animal care of the Nijmegen Animal Experiments Committee.

The murine melanoma cell line B16F10 (ATCC) was cultured in complete medium (MEM, 5% fetal bovine serum (Greiner Bio-one), 100 U/ml penicillin G sodium and 100 µg/ml streptomycin (Pen/Strep), MEM sodium pyruvate (1mM), NaHCO₃, MEM vitamins, MEM non-essential amino acids (all from Gibco), 20 µM β-mercaptoethanol (β -ME)).

### Tumor model and cryosurgery

Tumor cells were suspended in a mixture of PB S and Matrigel (2:1), and 0.5*10⁶ cells in a total volume of 50 µl were injected s.c. at the right femur. When tumor diameters measured between 6-8 mm (generally at day 9-10) they were randomly assigned to treatment groups. Cryo ablation (Cryo) was performed under isoflurane/O₂/N₂O anesthesia using a liquid nitrogen cryoablation system (CS76, Frigitronics, Shelton, CT) of which the tip is cooled by a continuous flow of circulating liquid nitrogen. During 2 treatment cycles of freezing and thawing the tumor was macroscopically frozen, while leaving surrounding healthy tissue intact. To monitor the induction of long-lasting tumor protection, mice were re-challenged with 15*10³ B160VA or B16F10 cells 40 days after cryo ablation. Re-challenges were injected in 100 µl PBS s.c. on the right flank. Mice were sacrificed when tumor volume exceeded 1000 mm³ or when tumors brake through the skin barrier.

### Adjuvant injection

CpG 1668 ('5-TCCATGACGTTCCTGATGCT-3') with total phosphorothioate-modified backbone was purchased from Sigma Genosys (Haverhill, UK). CpG was injected in PBS peri-tumorally (p.t., 30 µg divided over 2 injections of 10 µl lining the ablated tumor). The following adjuvants were used (all supplied by Intervet BV, Boxmeer): a water-in-oil emulsion based on mineral oil (Marcol 52) (1) and a water-in-oil emulsion based on non-mineral oil (Miglyol 840) (1) ; an oil-in-water emulsion using mineral oil, an oil-in-water emulsion using squalene (2); and an oil-in-water emulsion using vitamin E acetate (3); Matrix C 750 µg/ml (Isconova); Quil A Saponin (Brenntag) 500 µg/ml; aluminum hydroxide (Brenntag) 0.75% (w/v) ; or aluminum phosphate

(Brenntag) 0.75% (w/v) . In this article, the two water-in-oil emulsions were mixed at a 1:1 ratio and the three oil-in-water emulsions were mixed at a 1:1:1 ratio. The aluminum-based adjuvants were used mixed at a 1:1 ratio, but also separately. All non-microbial adjuvants (or mixes of them) were injected p.t. (40 µl divided over 2 injections of 20 µl, spatially separated from the CpG-ODN injections). All injections were done within 30 min. after ablation.

(1 : Jansen et al, Vaccine, 23, 1053-1060, 2005, 2 : O'Hagan Expert Re. Vaccines, 6, 669-710, 2007, 3: Rijke et al, in Adv. Avian Immunol. Res. Eds. T.F. Davison, N. Bumstead and P. Kaiser 265-271, 1995)

### Statistical analyses

Kaplan Meier survival curves were analyzed using a log rank test.

### Results:

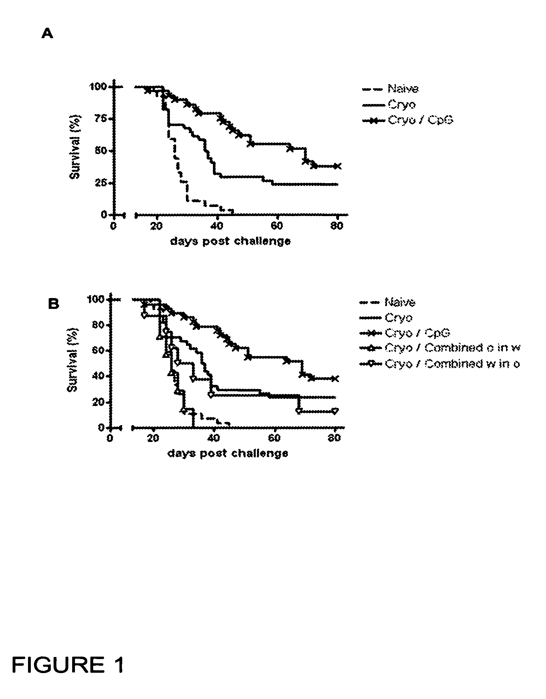

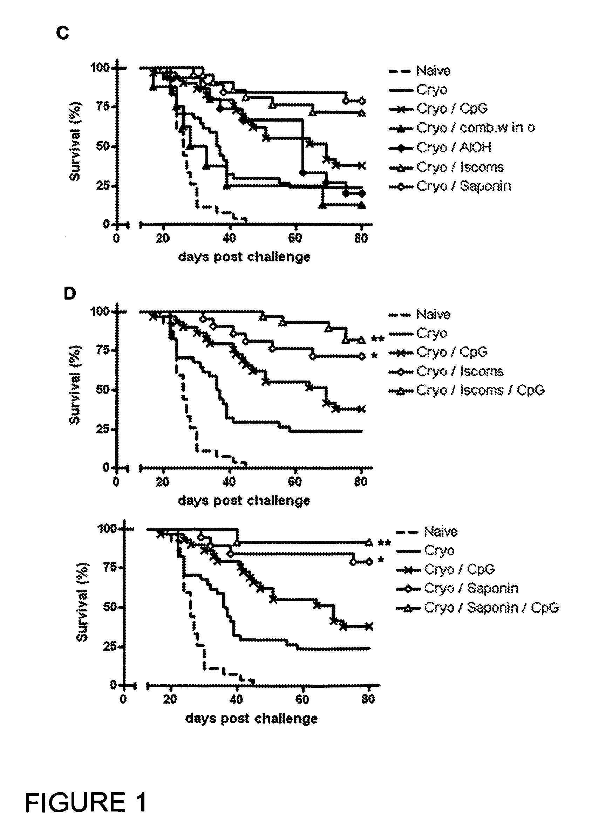

As follows clearly from the graphs of figure 1, the combination of tumor destruction and the administration of CpG ODN as immunopotentiator leads to a sub-50% survival rate after 80 days. Moreover, there is no significant leveling off of the survival curve (Fig. 1a).

The combination of tumor destruction and the administration of the oil-in-water, the water-in-oil or the AIOH adjuvants as immunopotentiator all led to less protection (Fig. lb and 1c).

However, the combination of tumor destruction and the administration of saponin, be it in the form of QuilA or as empty immune stimulating complexes, as immunopotentiator leads to an impressive survival rate of > 75% after 80 days. Moreover, in this case there is significant leveling off of the survival curve (Fig. 1c).

The combination of tumor destruction and the combined administration of CpG and saponin, be it in the form of QuilA or as empty immune stimulating complexes, as immunopotentiator leads to an even higher survival rate of > 90% after 80 days and a very strong leveling off of the survival curve (Fig. 1d).

### Legend to the figures

Figure 1. Potent anti-tumor immunity following ablation combined with CpG-ODN and saponin-based adjuvants. Established B16F 10 tumors on the right femur were treated with cryo ablation alone, in combination with CpG, or in combination with the indicated non-microbial adjuvants. Forty days later, naive and tumor-free mice received a s.c. re-challenge with tumor cells (15.000 B16F10 cells) at the flank. The tumor size was monitored every 2-4 days.
(A) Kaplan-Meier survival curve demonstrating limited protection from tumor outgrowth after ablation alone, or in combination with CpG-ODN.
(B) Survival curves demonstrating limited or no protection from tumor outgrowth after ablation alone, or in combination with the mixed oil-in-water, water-in-oil or aluminum adjuvants.
(C) Survival curve demonstrating relative protection from tumor outgrowth after ablation alone, or in combination with the indicated (mixed) adjuvants. The saponin-based adjuvants show the most potent protection.
(D) Survival curve demonstrating additional protection when ablation in combination with the saponin-based adjuvants is combined with CpG-ODN co-administration. *= p<0.05 compared to cryo, **=p<0.001 compared to cryo/CpG. Comparable data were obtained in three independent experiments.

## Claims

1. Pharmaceutical composition for use in *in situ* tumor-destruction therapy comprising the steps of tumor destruction and administration of an immunostimulating amount of an immunopotentiator wherein the pharmaceutical composition comprises the immunopotentiator and wherein said immunopotentiator is a saponin.

2. Pharmaceutical composition for use according to claim 1, wherein said saponin comprises at least one of the following components: QS-7, QS-17, QS-18, QS-21, QuilA, Vax Sap, SuperSap, GPI-0100 or QP UF 1000.

3. Pharmaceutical composition for use according to claim 1, wherein the saponin is in the form of an empty immune stimulating complex.

4. Pharmaceutical composition for use according to any of claims 1-3, wherein the pharmaceutical composition in addition it comprises a non-methylated cytidyl guanosyl oligodeoxynucleotide (CpG ODN).

5. Pharmaceutical composition for use according to any of claims 1-4 , **characterized in that** said steps are in the following order:
c. destruction of the tumor
d. administration of an immunopotentiator

6. Pharmaceutical composition for use according to claim 5, **characterized in that** the said step of administration of an immunopotentiator follows within 24 hour after tumor destruction.

7. Pharmaceutical composition for use according to claim 5, **characterized in that** the said step of administration of an immunopotentiator follows within 12 hour after tumor destruction.

8. Pharmaceutical composition for use according to claim 5, **characterized in that** the said step of administration of an immunopotentiator follows within 6 hour after tumor destruction.

9. Pharmaceutical composition for use according to any of claims 1-4 , **characterized in that** said steps are:
c. peri-operative administration of an immunopotentiator
d. destruction of the tumor

10. Pharmaceutical composition for use according to any of claims 1-4, wherein the site of administration of the immunopotentiator is intravenous, subcutaneous in the draining area of the neoplastic mass, peri-tumoral or intra-tumoral, in that order of increasing preference.

11. Use of a pharmaceutical composition according to any of claims 1-4, in the manufacture of a medicament for the treatment of a mammal suffering from cancer wherein the mammal has been subjected to tumor destruction.

12. Use of a pharmaceutical composition according to any of claims 1-4, in the manufacture of a medicament for peri-operative administration, for the treatment of a mammal suffering from cancer wherein the mammal will be or has been subjected to tumor destruction.

## Patentansprüche

1. Pharmazeutische Zusammensetzung für die Verwendung in der in-situ-Tumordestruktionstherapie, umfassend die Schritte der Tumordestruktion und die Verabreichung einer immunstimulierenden Menge eines Immunpotentiators, wobei die pharmazeutische Zusammensetzung den Immunpotentiator umfasst und wobei es sich bei dem Immunpotentiator um ein Saponin handelt.

2. Pharmazeutische Zusammensetzung für die Verwendung nach Anspruch 1, wobei das Saponin mindestens eine der folgenden Komponenten umfasst: QS-7, QS-17, QS-18, QS-21, QuilA, Vax Sap, SuperSap, GPI-0100 oder QP UF 1000.

3. Pharmazeutische Zusammensetzung für die Verwendung nach Anspruch 1, wobei das Saponin in Form eines leeren immunstimulierenden Komplexes vorliegt.

4. Pharmazeutische Zusammensetzung für die Verwendung nach einem der Ansprüche 1-3, wobei sie zusätzlich ein nicht methyliertes Cytidylguanosyloligodesoxynukleotid (CpG ODN) umfasst.

5. Pharmazeutische Zusammensetzung für die Verwendung nach einem der Ansprüche 1-4, **dadurch gekennzeichnet, dass** die Schritte in der folgenden Reihenfolge vorgenommen werden:
c. Destruktion des Tumors
d. Verabreichung eines Immunpotentiators.

6. Pharmazeutische Zusammensetzung für die Verwendung nach Anspruch 5, **dadurch gekennzeichnet, dass** der Schritt des Verabreichens eines Immunpotentiators innerhalb von 24 Stunden nach der Tumordestruktion erfolgt.

7. Pharmazeutische Zusammensetzung für die Verwendung nach Anspruch 5, **dadurch gekennzeichnet, dass** der Schritt des Verabreichens eines Immunpotentiators innerhalb von 12 Stunden nach der Tumordestruktion erfolgt.

8. Pharmazeutische Zusammensetzung für die Verwendung nach Anspruch 5, **dadurch gekennzeichnet, dass** der Schritt des Verabreichens eines Immunpotentiators innerhalb von 6 Stunden nach der Tumordestruktion erfolgt.

9. Pharmazeutische Zusammensetzung für die Verwendung nach einem der Ansprüche 1-4, **dadurch gekennzeichnet, dass** es sich bei den Schritten um Folgende handelt:
c. perioperative Verabreichung eines Immunpotentiators
d. Destruktion des Tumors.

10. Pharmazeutische Zusammensetzung für die Verwendung nach einem der Ansprüche 1-4, wobei die Verabreichungsstelle des Immunpotentiators mit steigender Bevorzugung intravenös, subkutan in die Drainagezone der Neoplasmamasse, peritumoral oder intratumoral ist.

11. Pharmazeutische Zusammensetzung für die Verwendung nach einem der Ansprüche 1-4 in der Herstellung eines Arzneimittels für die Behandlung eines an Krebs leidenden Säugers, wobei bei dem Säuger eine Tumordestruktion erfolgt ist.

12. Pharmazeutische Zusammensetzung für die Verwendung nach einem der Ansprüche 1-4 in der Herstellung eines Arzneimittels für die perioperative Verabreichung, für die Behandlung eines an Krebs leidenden Säugers, wobei bei dem Säuger eine Tumordestruktion erfolgen wird oder erfolgt ist.

## Revendications

1. Composition pharmaceutique pour utilisation dans une thérapie de destruction de tumeur *in situ* comprenant les étapes de destruction de tumeur et d'administration d'une quantité immunostimulante d'un immunopotentialisateur, la composition pharmaceutique comprenant l'immunopotentialisateur et ledit immunopotentialisateur étant une saponine.

2. Composition pharmaceutique pour utilisation selon la revendication 1, dans laquelle ladite saponine comprend au moins un des composants suivants : QS-7, QS-17, QS-18, QS-21, QuilA, Vax Sap, SuperSap, GPI-0100 ou QP UF 1000.

3. Composition pharmaceutique pour utilisation selon la revendication 1, dans laquelle la saponine est sous la forme d'un complexe immunostimulant vide.

4. Composition pharmaceutique pour utilisation selon l'une quelconque des revendications 1 à 3, la composition pharmaceutique comprenant en outre un cytidyl-guanosyl-oligodésoxynucléotide non méthylé (CpG-ODN).

5. Composition pharmaceutique pour utilisation selon l'une quelconque des revendications 1 à 4, **caractérisée en ce que** lesdites étapes sont dans l'ordre suivant :
c. destruction de la tumeur
d. administration d'un immunopotentialisateur.

6. Composition pharmaceutique pour utilisation selon la revendication 5, **caractérisée en ce que** ladite étape d'administration d'un immunopotentialisateur est conduite dans les 24 heures suivant la destruction de tumeur.

7. Composition pharmaceutique pour utilisation selon la revendication 5, **caractérisée en ce que** ladite étape d'administration d'un immunopotentialisateur est conduite dans les 12 heures suivant la destruction de tumeur.

8. Composition pharmaceutique pour utilisation selon la revendication 5, **caractérisée en ce que** ladite étape d'administration d'un immunopotentialisateur est conduite dans les 6 heures suivant la destruction de tumeur.

9. Composition pharmaceutique pour utilisation selon l'une quelconque des revendications 1 à 4, **caractérisée en ce que** lesdites étapes sont :
c. administration périopératoire d'un immunopotentialisateur
d. destruction de la tumeur.

10. Composition pharmaceutique selon l'une quelconque des revendications 1 à 4, le site d'administration de l'immunopotentialisateur étant intraveineux, sous-cutané dans la zone de drainage de la masse néoplasique, péritumoral ou intratumoral, dans cet ordre de préférence croissante.

11. Utilisation d'une composition pharmaceutique selon l'une quelconque des revendications 1 à 4, dans la fabrication d'un médicament pour le traitement d'un mammifère souffrant d'un cancer, le mammifère ayant été soumis à une destruction de tumeur.

12. Utilisation d'une composition pharmaceutique selon l'une quelconque des revendications 1 à 4, dans la fabrication d'un médicament pour administration périopératoire, pour le traitement d'un mammifère souffrant d'un cancer, le mammifère devant être ou ayant été soumis à une destruction de tumeur.
